# EUROPEAN PATENT SPECIFICATION

(11) **EP 4 178 584 B1**
(45) Date of publication and mention of the grant of the patent: **25.10.2023**
(21) Application number: 21793863.8
(22) Date of filing: 05.10.2021
(51) Int. Cl.: A61K 31/704, A61K 31/675, A61P 35/00, A61K 9/14, A61K 45/06, A61K 9/00

(54) **HYDROXYAPATITE COMPOSITION COMPRISING ZOLEDRONIC ACID AND DOXORUBICIN FOR TREATING CANCER**
HYDROXYAPATIT-ZUSAMMENSETZUNG MIT ZOLEDRONSÄURE UND DOXORUBICIN ZUR BEHANDLUNG VON KREBS
COMPOSITION D'HYDROXYAPATITE COMPRENANT DE L'ACIDE ZOLÉDRONIQUE ET DE LA DOXORUBICINE POUR LE TRAITEMENT DU CANCER

(30) Priority: 05.10.2020 SE 2051170
(43) Date of publication of application: 17.05.2023
(73) Proprietor: Moroxite T AB, 222 35 Lund (SE)
(72) Inventor: LIDGREN, Lars, 298 31 TOLLARP (SE); TÄGIL, Magnus, 298 31 TOLLARP (SE); RAINA, Deepak, 298 31 TOLLARP (SE); YANG, Liu, 298 31 TOLLARP (SE)
(74) Representative: Zacco Sweden AB
(86) International application number: PCT/EP2021/077420
(87) International publication number: WO 2022/073990

(56) References cited:
- WO-A1-2019/206677
- WO-A2-2013/008240
- US-A1- 2009 215 729
- PIGNATELLO ROSARIO ET AL: "Synthesis and Biological Evaluation of a New Polymeric Conjugate and Nanocarrier with Osteotropic Properties", JOURNAL OF FUNCTIONAL BIOMATERIALS, vol. 3, no. 1, 19 January 2012 (2012-01-19), pages 79-99, XP055880989, DOI: 10.3390/jfb3010079 Retrieved from the Internet: URL:https://www.ncbi.nlm.nih.gov/pmc/artic les/PMC4031017/pdf/jfb-03-00079.pdf>
- YUAN YE ET AL: "A multiple drug loaded, functionalized pH-sensitive nanocarrier as therapeutic and epigenetic modulator for osteosarcoma", SCIENTIFIC REPORTS, vol. 10, no. 15497, 23 September 2020 (2020-09-23), pages 1-11, XP055857230, DOI: 10.1038/s41598-020-72552-z Retrieved from the Internet: URL:https://www.nature.com/articles/s41598 -020-72552-z.pdf>
- AKOURY ELIE ET AL: "Anti-Tumor Effects of Low Dose Zoledronate on Lung Cancer-Induced Spine Metastasis", JOURNAL OF CLINICAL MEDICINE, vol. 8, no. 8, 14 August 2019 (2019-08-14) , page 1212, XP055881159, DOI: 10.3390/jcm8081212
- DEEPAK BUSHAN RAINA ET AL: "A Biphasic Calcium Sulphate/Hydroxyapatite Carrier Containing Bone Morphogenic Protein-2 and Zoledronic Acid Generates Bone", SCIENTIFIC REPORTS, vol. 6, no. 1, 18 May 2016 (2016-05-18), XP055457462, DOI: 10.1038/srep26033
- LIU YANG ET AL: "Sustained and controlled delivery of doxorubicin from an in-situ setting biphasic hydroxyapatite carrier for local treatment of a highly proliferative human osteosarcoma", ACTA BIOMATERIALIA, ELSEVIER, AMSTERDAM, NL, vol. 131, 13 July 2021 (2021-07-13), pages 555-571, XP086746921, ISSN: 1742-7061, DOI: 10.1016/J.ACTBIO.2021.07.016 [retrieved on 2021-07-13]

## Description

### Technical field

Present invention relates to a novel composition and administration mode of a particulate material, i.e. micro and/or nano sized hydroxyapatite (HA) and an anthracycline being doxorubicin and a bisphosphonate, being zoledronic acid. Present invention relates to use of the novel compositions according to the invention for treatment of cancer and in particular solid tumours.

### Background of the invention

Both osteosarcoma and Ewing's sarcoma are highly malignant tumours, usually affecting children and adolescents. Chemotherapy has dramatically increased the prognosis for the affected individuals both pre and postoperatively but for non-responders the options are few. For osteosarcoma, methotrexate, doxorubicin (Adriamycin), and cisplatin (MAP) form the backbone of standard treatment protocol. However, approximately 40% patients show a poor response to chemotherapy, with a 5-year survival of 45% to 55%. A recent clinical study conducted by EURAMOS-1 compared postoperative MAP with MAP plus ifosfamide and etoposide (MAPIE) in patients with a poor response. No significant benefit was achieved by using MAPIE and its administration was associated with increased toxicity without improving event-free survival. For Ewing's sarcoma (ES), induction chemotherapy with vincristine, doxorubicin, and cyclophosphamide (VIDE) is now considered the standard of care in Europe, whereas compressed vincristine, doxorubicin, and cyclophosphamide plus ifosfamide and etoposide (VDC-IE) is the North American standard. In a study conducted by Italian Sarcoma Group (ISP) and Scandinavian Sarcoma Group (SSG), they showed 51% (154 out of 300) Ewing's sarcoma patients had a poor response to chemotherapy. For poor response patients, five-year event-free survival (EFS) was about 33% under conventional chemotherapy. A review from E Hanafy et al. concluded that no strong evidence exists that intensifying systemic chemotherapy in poor responders results in better outcome or at least still needs further validation. Adding more systemic drugs does not improve outcome and rather, other strategies to efficiently deliver the drugs to the target sites are thus necessary.

Various ways of improving drug delivery to the target site have been explored within the last three decades, which can be divided into passive or active target delivery based on the mechanism. Passive target delivery is based on enhanced permeability and retention effect (EPR effect) by loading drugs on a nano-size carrier such as Doxil, for which doxorubicin (Dox) is encapsuled in liposome. However, limited evidence base to support the superiority of the liposomal doxorubicin compared to the conventional doxorubicin in efficacy when it comes to patients, with the only benefit to reduced cardiotoxicity. Active target delivery is trying to decorate surface of the nanocarrier with ligands binding to the receptors overexpressed on tumour cells, which has been considered to significantly increase the quantity of drug delivered to the target cell compared to free drug or passively targeted nano-systems. However, a comprehensive review paper from Stefan Wilhelm published on Nature Reviews Materials reported only 0.7% (median) of the administered nanoparticle dose is found to be delivered to a solid tumour. Another review paper from Kinam Park published on Journal of Controlled Release has the same opinion that only a very small fraction (<5%) of the total administered formulation is actually delivered to the intended target site. Moreover, the fabrication processes of these nano-systems are highly complicated regardless of passive or active strategy. Thus, nano particles as carrier for tumour drugs has been largely unsuccessful in clinical translation mainly due to insufficient carriers and delivery at the target site.

Pignatello Rosario et al.: "Synthesis and Biological Evaluation of a New Polymeric Conjugate and Nanocarrier with Osteotropic Properties", Journal of functional biomaterials, vol. 3, no. 1, 2012, pages 79-99, relates to a bone-seeking (osteotropic) drug delivery systems (ODDS) representing a solution for targeting different types of drugs to the bones. In particular, anticancer and antibacterial agents could take advantage of such therapeutic strategy. Moreover, the document relates to a osteotropic biomaterial, based on the conjugation of a poly(lactide-co-glycolide) (PLGA) with the bisphosphonate drug alendronate (PLGA-ALE).

Yuan Ye et al.: "A multiple drug loaded, functionalized pH-sensitive nanocarrier as therapeutic and epigenetic modulator for osteosarcoma", Scientific Reports, vol. 10, no. 15497, 2020, pages 1-11, relates to a multiple drug loaded, functionalized pH-sensitive nanocarrier as therapeutic and epigenetic modulator for osteosarcoma. The document further relates to a novel organic nanoparticle was devised conjugated with bisphosphonate zoledronic acid which has an affinity for bone tissues. Moreover, the nanoparticle was loaded with multiple anti-cancer drugs like gemcitabine and epirubicin. The nanoparticles were characterized by microscopic analysis, entrapment and loading efficiencies, bone affinity studies, in-vitro release studies, cytotoxicity studies and finally in-vivo tumour regression studies.

WO 2013/008240 relates to liposomes comprising a membrane and an intrahposomal aqueous water phase, the membrane comprising at least one liposome forming lipid and the intrahposomal aqueous water phase comprises a salt of a bisphosphonate together with an amphipathic weak base agent (PLAD).

US 2009/215729 relates to a pharmaceutical compositions and methods related to the prevention and treatment of primary tumours and metastatic, malignant or spreading cancers by selectively targeting cancer associated myeloid derived cells by the targeted delivery of a bisphosphonate formulated with a non-liposomal particle carrier.

Akoury Elie et al: "Anti-Tumor Effects of Low Dose Zoledronate on Lung Cancer- Induced Spine Metastasis", Journal of Clinical Medicine, vol. 8, no. 8, 2019, page 1212, relates to evaluating the effects of lower doses zoledronate (Zol) on lung cancer and lung cancer-induced bone metastasis cells over a longer time period. Human lung cancer (HCC827) and three bone metastases secondary to lung cancer (BMLI, BML3 and BML4) cells were treated with Zol at 1, 3 and 10 µM for 7 days and then assessed for cell proliferation, migration, invasion and apoptosis. Low Zol treatment significantly decreased cell proliferation (1, 3 and 10 µM), migration (3 and 10 µM) and invasion (10 µM) while increasing apoptosis (10 µM) in lung cancer and metastatic cells. The data exploits the potential of using low doses Zol for longer treatment periods and reinforces this approach as a new therapeutic regimen to impede the development of metastatic bone cancer while limiting severe side effects following high doses of systemic drug treatment.

Deepak Bushan Raina et al: "A Biphasic Calcium Sulphate/Hydroxyapatite Carrier Containing Bone Morphogenic Protein-2 and Zoledronic Acid Generates Bone", Scientific Reports, vol. 6, no. 1, 2016, relates to properties of a biphasic, calcium sulphate and hydroxyapatite ceramic material, containing a combination of recombinant human bone morphogenic protein-2 (rhBMP-2) to induce bone, and zoledronic acid (ZA) to delay early resorption.

WO 2019/206677 relates to a method of administering one or more pharmaceutical compounds to a subject in need thereof. The document further relates to the use of a mono or biphasic material, such as e.g. a ceramic material in combination with administration of one or more pharmaceutical compounds.

Hydroxyapatite (HA), a main component of calcified bone (70% inorganic mineral), has been successfully synthesized and approved for clinical usage.

Recently, the inventors of present invention found doxorubicin (Dox) could bind to nano-size synthetic hydroxyapatite (HA). Functionalized nano HA could facilitate delivery of drugs intracellularly, which in turn could enhances the cytotoxic effect. Combining systemic Dox and zoledronic acid (ZA) together has indicated a synergistic anti-tumour effect on subcutaneous breast tumour in mice. Present invention relates i.a. to a completely novel composition and treatment regimen which entails combining Dox and ZA using a carrier containing nano HA for local delivery and showed increased killing of tumour cells. This presents a significant clinical step forward and a major contribution to the art which overcomes the above discussed shortcomings. The findings of these aspects will be further elaborated upon below and in the following text.

### Summary of the invention

Present invention relates to a novel composition comprising a particulate material. The particulate material may be in various size particle sizes such as in micro sized particles. In another aspect, the particles may be in nano sized particles. In yet a further aspect, the particulate material may comprise a mixture of micro size and nano sized particles. The particulate material may be hydroxyapatite (HA).

The invention further relates to use of one or more drugs commonly used in treatment of therapy of cancer or tumour related diseases. In one aspect, the invention relates to anthracyclines, which may be drugs extracted from *Streptomyces spp.* According to the invention, the anthracycline is doxorubicin.

In another aspect, present invention relates to one or more compounds capable of binding to apatite in any binding mode. In one aspect, the invention relates to a combination of at least two compounds capable of binding to calcium apatite.

The above mentioned compounds or agents for use in the treatment of bone related tumour type selected from osteosarcoma (OS) or osteogenic sarcoma (OGS).

Furthermore, the invention relates to use of a bisphosphonate selected from zoledronic acid. The bisphosphonate may also be a salt wherein a radioactive compound is present or a radioactive compound as ⁹⁹Tc or ²²³Ra or strontium or samarium.

Present invention also enables a novel mode of administration.

Thus, the invention enables a novel administration wherein a subject in need of a treatment is administered the particulate material which may be e.g. HA (hydroxyapatite). Thereafter, the anthracyclines and bisphosphonate are administered to the subject in any order or simultaneously.

Moreover, the particulate material, for use according to the invention, onto which the one or more anthracyclines are absorbed, or otherwise bound to the particulate material, is administered to a subject. Said composition (HA+anthracyclines) is administered to a subject after which one or more bisphosphonates is/are administered.

Furthermore, the particulate material, for use according to the invention, onto which the one or more bisphosphonates are absorbed or otherwise bound to the particulate material is administered to a subject. Said composition (HA+bisphosphonates) is administered to a subject after which one or more anthracyclines is/are administered.

Alternatively, the particulate material, for use according to the invention, onto which the one or more bisphosphonates and one or more anthracyclines are absorbed or otherwise bound to the particulate material and is subsequently administered to a subject.

The inventors of present invention have surprisingly found that the combinations of particulate material and bisphosphonates and anthracyclines result in an increased cell toxicity in respect of tumorous cells.

### Brief description of the drawings

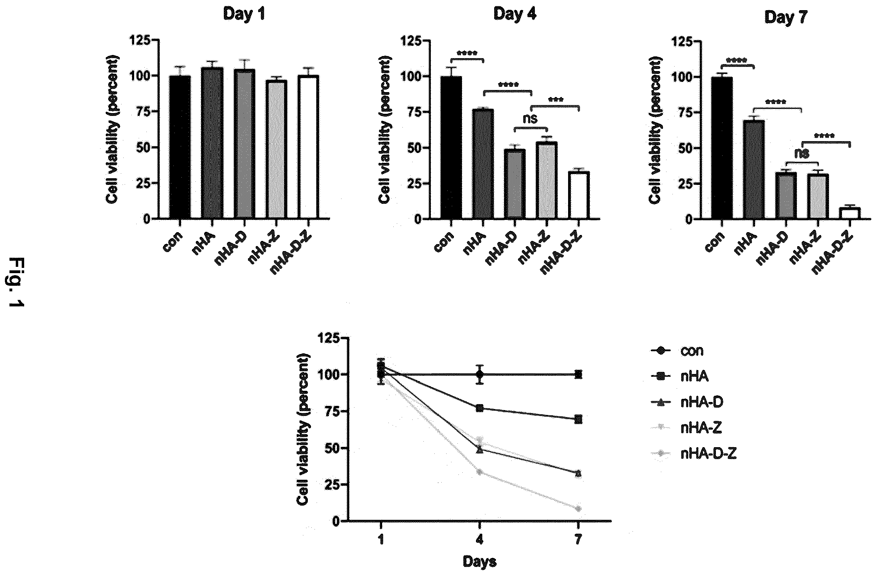

Fig. 1 illustrates the synergistic effect observed by the combination of particles with hydroxyapatite, doxorubicin and zoledronic acid with effect on cell viability observed at day 1, day 4 and day 7 respectively (after administration) in a head-to-head study of control (a control not containing any hydroxyapatite particles), nHA (nano sized particles of hydroxyapaptite), nHA-D (nano-sized particles of hydroxyapatite+doxorubicin), nHA+Z (nano-sized particles of hydroxyapatite+zoledronic acid), and nHA-D-Z (nano-sized particles of hydroxyapatite+doxorubicin+zoledronic acid).

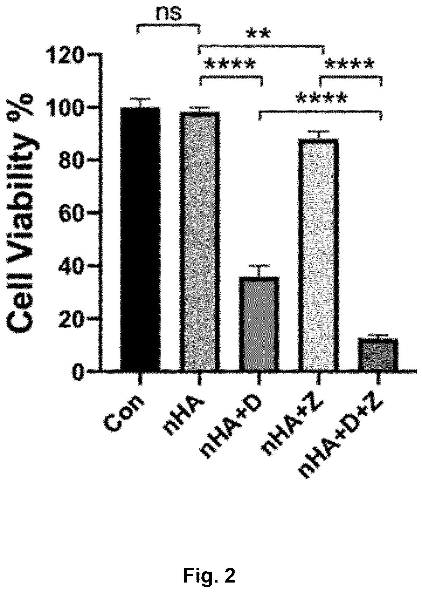

Fig.2 illustrates results from the MTT assay (assay employing enzymes that are capable of reducing the tetrazolium dye MTT 3-(4,5-dimethylthiazol-2-yl)-2,5-diphenyltetrazolium bromide to its insoluble formazan, which has a purple colour) showing the cytotoxic effect of DOX (doxorubicin) and ZA (zoledronic acid) functionalized nano-HA (hydroxyapatite) particles on A549 lung cancer cells. It is clearly illustrated that the DOX+ZA combination was the most toxic for the A549 cells.

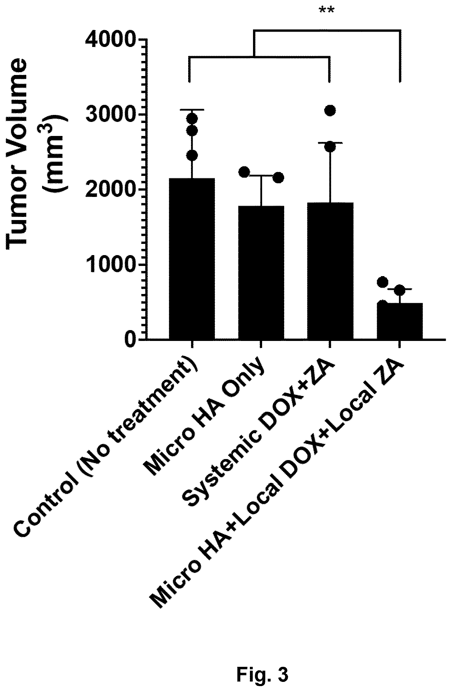

Fig. 3 illustrates the tumor volume in groups 1-3 was similar and no statistical differences could be noted. On the contrary, when DOX and ZA were locally delivered using the micro-HA particles, a strong effect on tumor growth inhibition was observed and the tumor volume in group 4 was significantly lower than all other groups (p<0.01).

### Detailed description of the invention

The inventors of present invention have surprisingly found that the combinations of particulate material and bisphosphonates and anthracyclines result in an increased cell toxicity in respect of tumorous cells.

Thus, present invention relates to a composition comprising a particulate material, and further comprising at least two pharmaceutical drug agents, and wherein the drug agents are anti-cancer drugs and/or drugs used in treatment of cancer, wherein the particulate material is hydroxyapatite in any crystalline form or configuration wherein the particles in the particulate material comprises or consist of particles in size range of 1 µm to 20 µm or in range of 20 nm to 120 nm,
and wherein one pharmaceutical drug agent is one or more bisphosphonates and one or more anthracyclines selected from zoledronic acid and doxorubicin respectively, or any suitable pharmaceutically acceptable salts or ester thereof.

In one aspect, the particulate material is hydroxyapatite (HA) in any form or configuration. The term "particulate" is meant to be understood as a material present in a fine particles or particle sizes specified herein.

One non-limiting example is hydroxyapatite. Hydroxyapatite (herein also, and interchangeably denoted as "HA"), is a naturally occurring mineral form of calcium apatite with the formula Ca₅(PO₄)₃(OH), but it is usually written Ca₁₀(PO₄)₆(OH)₂ to denote that the crystal unit cell comprises two entities.

The particulate material may be in any suitable size or size distribution. One non-limiting example is particles size ranges or distributions of less than about 100 nm, less than about 50 nm, less than about 45 nm, less than about 40 nm, less than 35 nm, less than about 30 nm, less than about 25 nm

In a further aspect, the particles may be 20 nm, such as e.g. about 25 nm, such as e.g. about 30 nm, such as e.g. about 35 nm, such as e.g. about 40 nm, such as e.g. about 45 nm, such as e.g. about 50 nm, such as e.g. about 100 nm, such as e.g. about 110 nm.

In another aspect, the particle size may be in range of about 30 nm to about 70 nm, such as e.g. about 50 nm.

In another aspect, the particle size may be in range of 20 nm to 80 nm, such as e.g. 30 nm to about 70 nm, or such as e.g. about 40 nm to about 60 nm, or 20 nm, or about 30 nm, or about 40 nm, or about 50 nm, or about 60 nm, or about 70 nm, or about 80 nm. In a further aspect, the particle size may be about 50 nm.

In a further aspect, the particle size may be in range of about 80 nm to 120 nm.

I another aspect, the particle size may be in range of about 100 nm to 120 nm, or about 110 nm.

I a further aspect, the particle size may be in range of less than 20 µm or less than 10 µm. In another aspect the microparticles may be in range of 1 µm to about 15 µm, or 1 µm to about 10 µm, or 1 µm, about 5 µm, about 10 µm, about 15 µm, or 20 µm.

In one aspect, the particles may be in range of e.g. about 1 µm to about 10 µm.

In another aspect, the particles may be in range of e.g. about 5 µm to about 15 µm, such as e.g. about 10 µm.

In yet a further aspect, the invention relates to a mixture of particles, The mixture of particles may comprise particles in range of 1 µm to about 15 µm, 1 µm to about 10 µm, or 1 µm, 5 µm, 10 µm, 15 µm, 20 µm, , and further comprise particles in range of, less than about 100 nm, less than about 50 nm, less than about 45 nm, less than about 40 nm, less than 35 nm, less than about 30 nm, less than about 25 nm,.

In one aspect, the invention relates to a mixture of particles in the range of e.g. 1 µm to about 10 µm and in range of about 40 nm to about 100 nm, or e.g. about 80 nm to 120 nm, or e.g. about 100 nm to 120 nm, or about 110 nm.

In one aspect, the invention relates to HA particles that may be in range of e.g. 1 µm to about 10 µm and in range of about 40 nm to about 100 nm, and HA particles of sizes of e.g. about 80 nm to 120 nm, or e.g. about 100 nm to 120 nm, or about 110 nm.

In a further aspect, the HA particles consist or comprises particles in size range of 5 µm to about 15 µm, and/or in range of about 30 nm to about 70 nm.

Present invention relates to use of one or more anthracyclines according to the invention, wherein the anthracycline is doxorubicin.

Present invention also relates to use of one or more bisphosphonates according to the invention, wherein the bisphosphonate is zoledronic acid. The bisphosphonate may also be a salt wherein a radioactive compound is present or a radioactive compound as ⁹⁹Tc or ²²³Ra or strontium or samarium.

Consequently, in one aspect, the bisphosphonate is zoledronic acid or any pharmaceutically acceptable salt or ester thereof.

In one aspect, the composition according to the invention may comprise a particulate material in size range of 20 nm to 80 nm, or about 50 nm, and further comprise doxorubicin and/or zoledronic acid or any pharmaceutically acceptable salt or ester thereof.

Consequently, and in one aspect, present invention relates to a composition comprising particulate hydroxyapatite in any configuration.

As mentioned herein, present invention also enables a novel method of administration of a subject in need thereof. Thus, the invention relates to a composition according to the invention for use in the treatment of cancer selected from bone related cancer, or osteosarcoma, wherein the composition is administered once every 7 days.

In one aspect, the one or more pharmaceutical active compounds for use according to the invention may be administered locally and/or systemically to a subject.

In one aspect, one pharmaceutical compound for use according to the invention may be administered locally and the other systemically. In a further aspect, both pharmaceutical compounds for use according to the invention may be administered systemically or locally.

In yet a further aspect, the one or more pharmaceutical compounds for use according to the invention may be administered in any order or simultaneously.

The one or more pharmaceutical compounds for use according to the invention may be administered by injection, or implantation, or alternatively in a tablet, capsule or any suitable formulation independently of each other. Alternatively, administration may be via the enteral route and/or the parenteral route.

In one aspect, the particulate material for use according to the invention, may be administered to a subject in need thereof. The administration may systemic or local, i.e. be administered to a desired site on the body of the subject. Thereafter, the anthracycline and/or the bisphosphonate may be administered locally or systemically in any order, and either separately or simultaneously.

In a further aspect, the particulate material for use according to the invention may be mixed with the bisphosphonate prior to being administered to a subject in need thereof. The administration may be systemic or local, i.e. be administered to a desired site on the body of the subject. Thereafter, the anthracycline may be administered locally or systemically to the subject.

In yet a further aspect, the particulate material for use according to the invention may be mixed with the anthracycline prior to being administered to a subject in need thereof. The administration may be systemic and local, i.e. be administered to a desired site on the body of the subject. Thereafter, the bisphosphonate may be administered locally or systemically to the subject.

In another aspect, the particulate material for use according to the invention may be mixed with the anthracycline and the bisphosphonate prior to being administered to a subject in need thereof. The administration may be systemic or local, i.e. be administered to a desired site on the body of the subject.

The inventors of present invention has surprisingly found that the long term effect is especially pronounced. Already after 4 days, the long term effect is particularly noticed and even more so 7 days after administration of the composition according to the invention.

In one aspect, the invention relates to administration once every 7 days.

In one aspect, the tumour may be e.g. osteosarcoma (OS) or also called osteogenic sarcoma (OGS).

As mentioned herein, it has surprisingly been found that a combination of a particulate material, one or more anthracyclines, and one or more bisphosphonates greatly improves treatment of tumours and consequently an unexpected increase in tumour cell death. This leads to a more efficient treatment regimen.

### Experimental section

Present invention will now be illustrated in the following examples.

As used throughout the description, "HA" is intended to mean hydroxyapatite. "ZA" is intended to mean zoledronic acid or a suitable pharmaceutically acceptable salt thereof. "Dox" is intended to mean doxorubicin or a suitable pharmaceutically acceptable salt thereof.

### Example 1

The inventors of present invention have surprisingly found that zoledronic acid (ZA) could increase the binding of doxorubicin (Dox) to nano-hydroxyapatite (nHA). In order to further investigate this, MG-63 cells were seeded in a 96-well plate and culturing for 24h to let cells attached to the bottom of the plate.The toxic effect of functionalized nHA was explored by setting up an experiment comprising thr MG-63 cells in 5 groups: 1. Control groups which was given normal medium; 2. Only nHA; 3. nHA-D (functionalized nHA with Dox); 4. nHA-Z (functionalized nHA with Dox); 5. nHA-D-Z (functionalized nHA with both Dox and ZA). Then check the cell viability on Day 1,4 and 7 after the point of administration. The result is illustrated in Fig. 1.

Preparation of functionalized nHA:
nHA: 10mg nHA+250µl PBS (average particle size of about 50 nm),
nHA-D: 10mg nHA+100µl Dox stock (170νg/ml)+150µl PBS,
nHA-Z: 10mg nHA+50µl ZA stock (0.8mg/ml)+200µl PBS,
nHA-D-Z: 10mg nHA+100µl Dox stock (170µg/ml)+50µl ZA stock, (0.8mg/ml)+100µl PBS,

After mixing, leave them in a sonication bath for 15 mins, then on a shaker for 48hrs. Centrifuge and throw the supernatant and wash the particles 4-5 times.

### Cell culture and treatment:

104 MG-63 cells were seeded in each well of 96-plate. After 24hs, treatments were given. For all the particle group, nHA were given at the concentration of 100µg/ml. Check cell viability on day 1, 4 and 7 by MTT cell proliferation/viability assay. As known to a person skilled in the art this is a colorimetric assay for assessing cell metabolic activity; MTT, a yellow tetrazole, is reduced to purple formazan in living cells. A solubilization solution like dimethyl sulfoxide (DMSO) is added to dissolve the insoluble purple formazan product into a colored solution. The absorbance of this colored solution can be quantified by measuring at a certain wavelength (usually between 500 and 600 nm) by a spectrophotometer. The degree of light absorption is dependent on the degree of formazan concentration accumulated inside the cell and on the cell surface, which could be used to measure cytotoxicity.

In more detail, we incubated MG-63 cells with different particles up to 7 days. The comparison was divided into 5 groups: 1, Control group without nHA; 2. Only nHA; 3. Functionalized nHA by Dox; 4. Functionalized nHA by ZA and 5. Functionalized by Dox and ZA. For all the particle groups, concentration of nHA was used as 100µg/ml. We could see there is no toxic effect on day 1 for all the groups. On day 4, 22.9% cells were killed by nHA. And nHA-D or nHA-Z could lead to more cell death, with 50.7% and 45.9% respectively. nHA-D-Z has the best cytotoxicity with 66.4% cells dead. The similar trend of toxic effect from each group was also seen on day 7 with 66.9% cells dead in nHA-D group and 68% for nHA-Z. For nHA-D-Z group, almost 91.6% cells have been cleared. Based on these data, we surprisingly found that Dox or ZA could bind to nHA and functionalized nHA has a strong toxic effect on tumor cells. When loading both drugs on nHA, we could achieve the best cytotoxicity.

### Example 2

In a further experiment, a human lung cancer cell line A549 (adenocarcinomic human alveolar basal epithelial cells) was used as a model for lung cancer. Following treatment groups were used: 1. No treatment (negative control), 2) Nano-HA alone, 3) Nano-HA+ DOX (0.44 µg/well), 4) Nano-HA+ZA (0.08 µg/well) and 5) Nano-HA+ DOX (0.44 µg/well) + ZA (0.08 µg/well). HA particles had an average size of about 50 nm. In all treatment groups receiving the nano particles, a total of 20 µg/well HA particles were given in each well. 104 A549 cells were seeded in each well of a 96-well plate. Cells were cultured with RPMI 1640 medium+10% serum+1 % antibiotics+ 1% glutamine. 24 h after seeding, medium was removed and new medium containing no nano particles (group 1) or nano particles alone or containing DOX or ZA or the combination (as described above in groups 2-5) were given to the cells. After a period of 48 h, the culture medium was replenished and new medium (similar to when the treatment was started) was provided to the cells for another 48 h. Finally, after a 4-day treatment period, viability of the cells was assessed using an MTT assay.

### Results:

The viability of controls (no treatment) or nano-HA alone treated cells was almost 100% after 4-days of treatment. Addition of only ZA to the nano-HA particles led to only 10-15% reduction in cell viability. Addition of DOX to the nano-HA particles induced cell death in about 65% cells. However, the combination of DOX and ZA functionalized nano-HA was the most cytotoxic combination inducing apoptosis in nearly 85% of the cells. The results are illustrated in Fig. 2.

It can thus be concluded that a combination of hydroxyapatite particles, an anthracycline and a bisphosphonate results in an unexpected synergistic effect leading to an unexpected high cytotoxicity.

### Example 3

A human osteosarcoma model was created in Athymic nude mice using 4×10⁶ 143B human osteosarcoma cells injected sub-cutaneously. After a period of 7-days, when the tumor was palpable and clearly visible, a small skin incision was made and a sharp incision in the core of the tumor was created. After this, the tumor was treated as follows: 1) No treatment (n=5), 2) Micro Hydroxyapatite (HA) particles (size approximately 10 microns) (n=5), 3) Systemic delivery of Doxorubicin (DOX) and Zoledronic acid (ZA), given sub-cutaneously to each animal at doses of 60 µg and 10 µg, respectively (n=6) and 4) Micro HA particles combined with local DOX and ZA at doses of 60 µg and 10 µg, respectively (n=6). In groups 2 and 4, pellets of micro-HA with or without cytostatics were inserted into the core of the tumor. The incision was sutured and 14-days later, the effect of the treatment was evaluated by measuring the tumor volume using a Vernier-Caliper. The results of the experiment are illustrated in Fig. 3.

## Claims

1. A composition comprising a particulate material, and further comprising at least two pharmaceutical drug agents, and wherein the drug agents are anti-cancer drugs and/or drugs used in treatment of cancer, wherein the particulate material is hydroxyapatite in any crystalline form or configuration wherein the particles in the particulate material comprises or consist of particles in size range of 1 µm to 20 µm or in range of 20 nm to 120 nm,
and wherein one pharmaceutical drug agent is one or more bisphosphonates and one or more anthracyclines selected from zoledronic acid and doxorubicin respectively, or any suitable pharmaceutically acceptable salts or ester thereof.

2. The composition according to claim 1, wherein at least one of the pharmaceutical drugs are capable of binding to the particulate material.

3. The composition according to any of the preceding claims, wherein the particles in the particulate material is a combination of different particles sizes in the micrometre range and in the nanometre range.

4. The composition according to any one of preceding claims, wherein the anthracycline is doxorubicin, and the bisphosphonate is zoledronic acid or any pharmaceutically acceptable salt or ester thereof, and the hydroxyapatite is present in particle size range of 20 nm to 80 nm, or 50 nm.

5. A composition according to any one of the preceding claims for use in the treatment of cancer selected from bone related cancer, or osteosarcoma, wherein the composition is administered once every 7 days.

6. The composition for use according to claim 5, wherein
(a) the doxorubicin is mixed with the particulate material prior to being administered to the subject or,
(b) the zoledronic acid, or any suitable pharmaceutically acceptable salts thereof, is mixed with the particulate material prior to being administered to the subject or,
(c) the doxorubicin, and the zoledronic acid, or any suitable pharmaceutically acceptable salts thereof, is mixed with the particulate material prior to being administered to the subject.

## Patentansprüche

1. Zusammensetzung, umfassend ein teilchenförmiges Material und ferner umfassend mindestens zwei pharmazeutische Arzneimittel, und wobei die Arzneimittel Anti-Krebs-Arzneimittel und/oder Arzneimittel sind, die bei der Behandlung von Krebs verwendet werden, wobei das teilchenförmige Material Hydroxyapatit in einer kristallinen Form oder Konfiguration ist, wobei die Teilchen in dem teilchenförmigen Material Teilchen im Größenbereich von 1 µm bis 20 µm oder im Bereich von 20 µm bis 120 µm umfassen oder daraus bestehen, und wobei ein pharmazeutisches Arzneimittel ein oder mehrere Bisphosphonate und ein oder mehrere Anthracycline, ausgewählt aus Zoledronsäure bzw. Doxorubicin, oder geeignete pharmazeutisch annehmbare Salze oder Ester davon sind.

2. Zusammensetzung nach Anspruch 1, wobei mindestens eines der pharmazeutischen Arzneimittel in der Lage ist, an das teilchenförmige Material anzubinden.

3. Zusammensetzung nach einem der vorhergehenden Ansprüche, wobei die Teilchen in dem teilchenförmigen Material eine Kombination verschiedener Teilchengrößen im Mikrometerbereich und im Nanometerbereich sind.

4. Zusammensetzung nach einem der vorhergehenden Ansprüche, wobei das Anthracyclin Doxorubicin und das Bisphosphonat Zoledronsäure oder ein pharmazeutisch annehmbares Salz oder ein Ester davon ist und der Hydroxylapatit in einem Teilchengrößenbereich von 20 nm bis 80 nm oder von 50 nm vorliegt.

5. Zusammensetzung nach einem der vorhergehenden Ansprüche zur Verwendung bei der Behandlung von Krebs, ausgewählt aus knochenbezogenem Krebs oder Osteosarkom, wobei die Zusammensetzung einmal alle 7 Tage verabreicht wird.

6. Zusammensetzung zur Verwendung nach Anspruch 5, wobei
(a) das Doxorubicin mit dem teilchenförmigen Material gemischt wird, bevor es dem Patienten verabreicht wird oder,
(b) die Zoledronsäure oder ein geeignetes pharmazeutisch annehmbares Salz davon mit dem teilchenförmigen Material gemischt wird, bevor es dem Patienten verabreicht wird, oder,
(c) das Doxorubicin und die Zoledronsäure oder geeignete pharmazeutisch annehmbare Salze davon vor dem Verabreichen an den Patienten mit dem teilchenförmigen Material gemischt werden.

## Revendications

1. Composition comprenant un matériau particulaire, et comprenant en outre au moins deux agents médicamenteux pharmaceutiques, et dans laquelle les agents médicamenteux sont des médicaments anticancéreux et/ou des médicaments utilisés dans le traitement du cancer, dans laquelle le matériau particulaire est de l'hydroxyapatite sous n'importe quelle forme cristalline ou configuration dans laquelle les particules dans le matériau particulaire comprennent ou sont constituées de particules dans une plage de tailles allant de 1 µm à 20 µm ou dans une plage allant de 20 nm à 120 nm, et dans laquelle un agent médicamenteux pharmaceutique est un ou plusieurs bisphosphonates et une ou plusieurs anthracyclines choisis parmi l'acide zolédronique et la doxorubicine respectivement, ou n'importe quels sels ou ester pharmaceutiquement acceptables appropriés de celui-ci.

2. Composition selon la revendication 1, dans laquelle au moins un des médicaments pharmaceutiques est capable de se lier au matériau particulaire.

3. Composition selon l'une quelconque des revendications précédentes, dans laquelle les particules dans le matériau particulaire sont une combinaison de différentes tailles de particules dans la plage du micromètre et dans la plage du nanomètre.

4. Composition selon l'une quelconque des revendications précédentes, dans laquelle l'anthracycline est la doxorubicine, et le bisphosphonate est l'acide zolédronique ou n'importe sel ou ester pharmaceutiquement acceptable de celui-ci, et l'hydroxyapatite est présente dans une plage de tailles de particules allant de 20 nm à 80 nm, ou 50 nm.

5. Composition selon l'une quelconque des revendications précédentes destinée à être utilisée dans le traitement d'un cancer choisi parmi le cancer lié aux os, ou l'ostéosarcome, dans laquelle la composition est administrée une fois tous les 7 jours.

6. Composition destinée à être utilisée selon la revendication 5, dans laquelle
(a) la doxorubicine est mélangée au matériau particulaire avant d'être administrée au sujet ou,
(b) l'acide zolédronique, ou n'importe quels sels pharmaceutiquement acceptables appropriés de celui-ci, est mélangé avec le matériau particulaire avant d'être administré au sujet ou,
(c) la doxorubicine et l'acide zolédronique, ou n'importe quels sels pharmaceutiquement acceptables appropriés de celui-ci, sont mélangés avec le matériau particulaire avant d'être administrés au sujet.
